# EUROPEAN PATENT SPECIFICATION

(11) **EP 3 257 386 B1**
(45) Date of publication and mention of the grant of the patent: **19.06.2019**
(21) Application number: 15881702.3
(22) Date of filing: 23.06.2015
(51) Int. Cl.: A24F 47/00

(54) **SMOKE GENERATOR AND ASSEMBLING METHOD THEREFOR**
RAUCHERZEUGER UND MONTAGEVERFAHREN DAFÜR
GÉNÉRATEUR DE FUMÉE ET PROCÉDÉ D'ASSEMBLAGE ASSOCIÉ

(30) Priority: 11.02.2015 CN 201520098744 U; 13.03.2015 CN 201510112097
(43) Date of publication of application: 20.12.2017
(73) Proprietor: CHINA TOBACCO YUNNAN INDUSTRIAL CO., LTD, Wuhua District Kunming Yunnan 650231 (CN)
(72) Inventor: MIAO, Mingming, Kunming Yunnan 650231 (CN); CHEN, Yongkuan, Kunming Yunnan 650231 (CN); TANG, Jianguo, Kunming Yunnan 650231 (CN); ZHENG, Xudong, Kunming Yunnan 650231 (CN); LEI, Ping, Kunming Yunnan 650231 (CN); SHANG, Shanzhai, Kunming Yunnan 650231 (CN); XIANG, Nengjun, Kunming Yunnan 650231 (CN); YUAN, Dalin, Kunming Yunnan 650231 (CN); SUN, Zhiyong, Kunming Yunnan 650231 (CN); YANG, Liu, Kunming Yunnan 650231 (CN); ZHU, Donglai, Kunming Yunnan 650231 (CN); LIAO, Xiaoxiang, Kunming Yunnan 650231 (CN); LI, Shoubo, Kunming Yunnan 650231 (CN); YANG, Ji, Kunming Yunnan 650231 (CN); ZHAO, Wei, Kunming Yunnan 650231 (CN)
(74) Representative: Maiwald Patent- und Rechtsanwaltsgesellschaft mbH
(86) International application number: PCT/CN2015/082079
(87) International publication number: WO 2016/127541

(56) References cited:
- EP-A1- 0 615 411
- WO-A1-95/27412
- CN-A- 102 326 869
- CN-A- 103 504 478
- CN-U- 202 456 410
- CN-U- 203 505 586
- CN-U- 203 505 592
- CN-U- 203 748 673
- US-A- 5 591 368
- US-A1- 2014 261 487

## Description

### Technical field

The present invention relates to the smoking device field, particularly to a method for assembling a smoke generator.

### Background Art

Smoking is not only harmful to health of smokers, but also pollutes surrounding environments. In order to reduce harms of smoking to the health of smokers and to reduce pollutions to the environments, a suction device for heating tobacco to produce smoke by a means of non-burning is developed at now. Compared to traditional cigarettes, the suction device can greatly reduce harmful substances (e.g., tar) that are sucked into human bodies by roasting solid tobacco at a lower temperature. For the above advantages, the novel suction device, as a novel smoking device, is gradually popular for cigarette consumers. However, in the prior art, heating structures in the interior of most novel cigarette smoking devices are not reasonably arranged. For example, a cylinder heater will repeatedly heat smoking articles so that the smoking articles will produce a burnt taste due to an overly high local temperature, thereby to influence consumer's mouth feels. There have been some heating devices that use common-end-type heaters in section, which are provided with a plurality of heating legs around the smoking articles. Upon each suction, only one of the heating legs is started to heat a sector of the smoking articles. However, such heaters in section usually are prepared by cutting a piece of metal sheet into a plurality of legs having a common end by a laser cutting process, and then rolling it into a common-end-type heater in section. Due to the design that the plurality of legs have a common end, when one heating leg is in failure, it is difficult to replace and repair it separately. Either the whole common-end-type heater in section has to be replaced, which can result in resource wastes, or it is used by putting up with the other heating legs, which will influence the application effects. In addition, for the accuracy issue of the laser cutting process, the consistence in the electric resistance of each leg is poor, for example, the variance of the electric resistance of each leg being about ±10%, which can influence the heating uniformity.

EP0615411A1 discloses a smoking system, the system is provided in which a replaceable cigarette containing tobacco flavor material is electrically heated by a set of electrical heater elements contained within a lighter to evolve tobacco flavors or other components in vapor or aerosol form for delivery to a smoker. The cigarette and lighter are adapted to provide air flow patterns through the smoking system such that air flows transversely into the cigarette. Such patterns improve aerosol and flavor delivery to the smoker and reduce the condensation of residual heater-region vapor/aerosol in the smoking system.

WO95/27412A1 discloses a tubular heater for use in an electrical smoking article. A cylindrical tube is provided of a mechanically strong and flexible electrical conductor such as a metal and has a plurality of separated regions. An electrically insulating layer such as a ceramic is applied on the outer surface except for one exposed portion. Electrically resistive heaters are then applied to the insulated regions and are electrically connected at one end to the underlying electrical conducting region. The electrical conductor is connected to the negative terminal of a power source. The other end of all the heaters are adapted to be connected to the positive terminal of the source. Accordingly, an electrically resistive heating circuit is formed wherein the tube serves as a common for all of the heating elements. The tubular heater can comprise an exposed end hub with a plurality of blades extending therefrom. Each blade can have an individual heater deposited thereon. Alternatively, every other blade can have a heater deposited thereon. The blades having no heaters function as barriers to minimize outward escape of generated vapors. Theses barrier blades also function as heat sinks for the heaters on adjacent blades.

Existing smoke suction devices available in the market usually include two assemblies of a supply power source and a smoke generator. Therein, the smoke generator has a complex structure, and processes for assembling it are also complex. Particularly, there are many heating parts in the smoke generator, and thus it is difficult to assure the reject ratio of finished products. Thus, the cost for industrial production of smoke generators is high.

Directed to the above defects, there is a need in the art for a method of quickly and effectively assembling a smoke generator.

### Summary of the Invention

The objective of the invention is to provide a smoke generator and assembling method for the smoke generator. The individual heating bodies in said smoke generator are independent of each other, and thus when one heating body is in failure, it can be replaced or repaired separately. Said assembling method has the advantages of simple and rapid process, high production efficiency and low reject ratio of finished products. In order to realize the above objective of the invention, the invention is provided with the following technical solution:
A first aspect of the invention is concerned with a smoke generator 100, comprising: a supporting pipe 10, a plurality of heating bodies 60 having a first end 63 and a second end 64 that are disposed in the supporting pipe 10, a first support 20 having an gas outlet 21 and a second support 30 having a gas inlet 31, the first support 20 and the second support 30 being respectively disposed at the two ends of the supporting pipe 10, and a plurality of positioning portions being disposed onto the first support 20 and the second support 30, wherein the first end 63 of each of the plurality of heating bodies 60 is disposed onto the first support 20 and bent onto the positioning portions of the first support 20, and the second end 64 of each of the plurality of heating bodies 60 is disposed on the second support 30; the second end 64 of the heating bodies 60 includes a first pin 61 and a second pin 62, in which the second pin 62 extends out of the second support 30, and the first pin 61 is bent into an arc and then fixed onto the positioning portions of the second support 30; the plurality of heating bodies 60 surrounds a cavity 11 that is adapted to be filled with smoking materials.

In a preferred embodiment, a plurality of through holes 12 for gas entrance are disposed onto the sidewalls of the supporting pipe 10.

A second aspect of the invention is concerned with a method for assembling a smoke generator, wherein the smoke generator 100 comprises: a supporting pipe 10, a plurality of heating bodies 60 having a first end 63 and a second end 64 that are disposed in the supporting pipe 10, a first support 20 having an gas outlet 21 and a second support 30 having a gas inlet 31, the first support 20 and the second support 30 being respectively disposed at the two ends of the supporting pipe 10, and a plurality of positioning portions being disposed onto the first support 20 and the second support 30, wherein the first end 63 of each of the plurality of heating bodies 60 is disposed onto the first support 20 and bent onto the positioning portions of the first support 20, and the second end 64 of each of the plurality of heating bodies 60 is disposed on the second support 30 and bent onto the positioning portions of the second support 30; the second end 64 of each heating body 60 of the smoke generator 100 is divided into a first pin 61 and a second pin 62 that are adaptable for electrical connection to the electrodes of the external supply power source, and the smoke generator 100 further comprises a non-conductive fastener 50 that is fastened in the gas inlet 31, and a base 40 that is engaged or sleeved onto the fastener 50; and the plurality of heating bodies 60 surrounds a cavity 11 that is adapted to be filled with smoking materials, the method for assembling the smoke generator comprising the steps of:
a). fixing a first support 20 and a second support 30 at two ends of a supporting pipe 10 respectively;
b). sleeving the supporting pipe 10 obtained in the step a) onto an assembling rod 91 adapted to pass through the supporting pipe 10;
c). inserting a first end 63 of the heating bodies 60 into a gap between the supporting pipe 10 and the assembling rod 91 along the end of the second support 30 until the first end 63 of the heating bodies 60 extends out of the end of the first support 20;
d). respectively bending two ends of the heating bodies 60 onto positioning portions of the first support 20 and second support 30 to fix the heating bodies 60; and the first pin 61 of the second end 64 of each heating body 60, after being bent, is positioned onto the position portion of the second support 30, and the plurality of the first pins 61 are brought into contact with each other, and the individual second pins 62 are intermittently fixed between the fastener 50 and the base 40;
e). successively fixing the individual heating bodies 60 along the circumferential direction of the supporting pipe 10 onto the first support 30 and the second support 30, so that the plurality of heating bodies 60 surround a cavity 11 that is adapted to be filled with solid smoking materials.

The smoke generator as involved in the invention is adapted to heat solid smoking materials (e.g., to heat solid tobacco materials) by the means of electric heating, to produce smoke for human suctions. The solid smoking materials may be either tobacco materials in a powder form or a particulate form, or tobacco articles that are processed to have a constant shape. In the method, when the smoke generator is in operation, a gas stream flows into the cavity 11 through the gas inlet, and then it is discharged from the cavity 11 via the gas outlet 21. Preferably, the supporting pipe 10 is made of metallic materials, and the heating bodies 60 are insulated from the supporting pipe 10. Preferably, a plurality of through-holes 12 for gas entrance are disposed on the side of the supporting pipe 10, and the through hole 12 passes through the cavity 11.

In a preferred embodiment, the assembling rod 91 is a part of an assembling tool, and the assembling tool comprises a substrate 92 and an assembling rod 91 disposed on the substrate 92.

Preferably, the heating bodies 60 are in a sheet form, and the number of the heating bodies 60 may be set to be more than three. The method for assembling the plurality of heating bodies may be conducted as follows: first of all, according to the step c), the heating bodies are assembled onto the supporting pipe 10 one by one; thereafter, according to the step d), each of the first ends 63 of the plurality of heating bodies 60 is bent and positioned onto the positioning portions, and then each of the first pins 61 of the second end 64 is bent and positioned on the positioning portions. Therein, the fastener 50 is used to fix the first pin 61 on each of the heating bodies 60 and to separate the first pins 61 from the second pins 62 to prevent false conductions. The base is used to intermittently fix the second pin of each heating body between the fastener and the base.

In a preferred embodiment, the positioning portion of the first support 20 or the second support 30 is a groove that is adapted to contain the bent first end 63 or bent second end 64 of the heating bodies 60. The positioning portion on the first end 63 is a first groove 22, and the positioning portion of the second end 64 is the second groove 32.

In a preferred embodiment, the smoke generator 100 further comprises a fixing cap 70 that engages the first support 20, and the method for assembling the smoke generator further comprises the step:
f). detaching the supporting pipe 10, further sleeving the supporting pipe 10 onto the assembling rod 91 after the fixing cap 70 is sleeved onto the assembling rod 91, and engaging the fixing cap 70 onto the first support 20 along the assembling rod 91 while the fixing cap 70 is pressured onto the first end 63 of the heating bodies 60, wherein, the fixing cap 70 is adapted to compactly press the first end of the heating bodies, after being bent, onto the positioning portion of the first support.

As compared to the prior art, the smoke generator and method for assembling the smoke generator according to the invention have the following advantageous effects:
1. The plurality of heating bodies as involved in the invention, along the length direction of a smoking article, are dispose in a cavity that contains the smoking article. The internal side of each heating body is attached to the smoking article, and has a shape adaptive to the outer surface of the smoking article. Furthermore, the above plurality of heating bodies are uniformly distributed along the circumferential direction of the cavity. In such a structure, not only each heating body can be in a uniform contact with the surface of the smoking article, thereby to homogenously transmit heat to the smoking article, but also the plurality of heating bodies can form a heater in a whole to homogenously radiate heat to the smoking article from the around thereof. Each of the heating bodies are separated from each other, and thus its operation can be separately controlled by controlling electric circuit. Thus, burnt taste as produced due to an overly high local temperature of the smoking article is prevented so that produced smoke is assured to have a good mouth feel. For the design that the plurality of heating bodies are separate from each other, once one heating body is in failure, it can be replaced or repaired separately of each other, thereby to avoid senseless write-off of the other heating bodies that are in normal operation. In addition, since the individual heating bodies in the invention are separate from each other, it can be molded in bulk production by conventional forging or casting processes. Thus, the expensive laser cutting process may be avoided, and this not only can reduce production coast, but also can decrease the fluctuation of the electrical resistance between heating bodies to be about ± 3%, thereby to increase the heating consistence.
2. In the assembling method according to the invention, since the smoke generator in the invention are installed with a plurality of positioning portions on both the first support and the second support respective disposed at the two ends of the smoke generator, the smoke generator positions and fixes the plurality of heating bodies via the first and second supports. The first end of the plurality of heating bodies is disposed on the first support and bent onto the positioning portion of the first support, and the second end is disposed on the second support and bent onto the positioning portion of the second support. Thereby, the plurality of heating bodies surrounds a cavity that is adapted to be filled with smoking materials. Thus, by the positioning with the positioning portions, the movement of the heating bodies may be prevented, so as to better decrease the reject ratio in the finished smoke generators. Furthermore, the bending disposition way can have the advantageous effects of a simple assembling structure and a high production efficiency.
3. In the assembling method according to the invention, the plurality of heating bodies in the smoke generator is disposed in the cavity along the length direction of the smoking article. The internal side of each heating body is attached to the smoking article, and has a shape adaptive to the outer surface of the smoking article. Furthermore, the above plurality of heating bodies are uniformly distributed along the circumferential direction of the cavity. In such a structure, not only each heating body can be in a uniform contact with the surface of the smoking article, thereby to homogenously transmit heat to the smoking article, but also the plurality of heating bodies can form a heater in a whole to homogenously radiate heat to the smoking article from the around thereof. Each of the heating bodies are separate from each other, and thus its operation can be controlled by controlling electric circuit. Thus, burnt taste as produced due to an overly high local temperature of the smoking article is prevented so that produced smoke is assured to have a good mouth feel. For the design that the plurality of heating bodies are separate from each other, once one heating body is in failure, it can be replaced or repaired separately of each other, thereby to avoid senseless write-off of the other heating bodies that are in normal operation. In addition, since the individual heating bodies in the invention are separate from each other, it can be molded in bulk production by conventional forging or casting processes. Thus, the expensive laser cutting process may be avoided, and this not only can reduce production coast, but also can decrease the fluctuation of the electrical resistance between heating bodies to be about ±3%, thereby to increase the heating consistence.

### Descriptions to the Drawings of the Invention

Fig. 1 is a graphic diagram for the smoke generator in the invention;
Fig. 2 is a sectional view for the smoke generator in the invention;
Fig. 3 is an exploded structural schematic view for the smoke generator in the invention in which the heating bodies are detached;
Fig. 4 is a structural schematic view for the heating bodies in the invention;
Fig. 5 is a structural schematic view for the supporting pipe with through holes disposed thereon in the invention.
Fig. 6 is a structural schematic view when the heating bodies are installed during the process of assembling the smoke generator.
Fig. 7 is a structural schematic view when one heating body is installed in the process for assembling the smoke generator.
Fig. 8 is a structural schematic view when the fastener is installed in the process for assembling the smoke generator.
Fig. 9 is a structural schematic view when the base is installed in the process for assembling the smoke generator.
Fig. 10 is a sectional view for illustrating the structure of the smoke suction device comprising the smoke generator according to the invention.

### Best Mode for Carrying Out the Invention

In order to more clearly illustrate the technical problems to be solved by the invention, and the technical solution and advantageous effects thereof, the invention is further described in detail by combining the following examples and the drawings. It should be understood that the specific examples as described below are only used for explaining the invention, but not for restricting the invention.

### Example 1

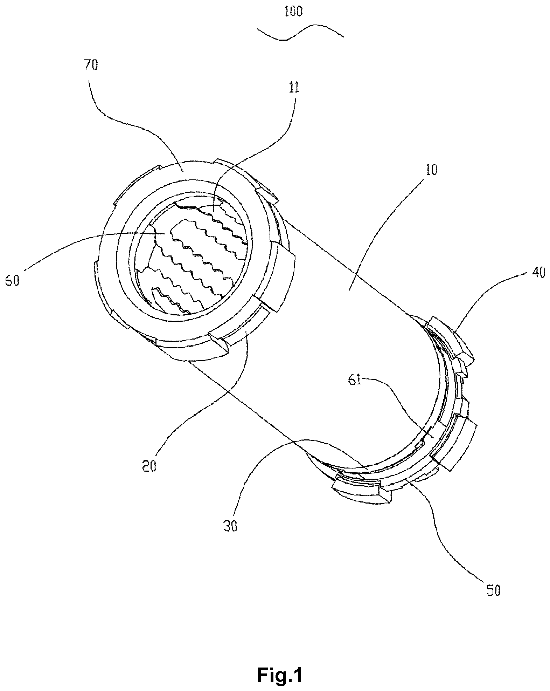

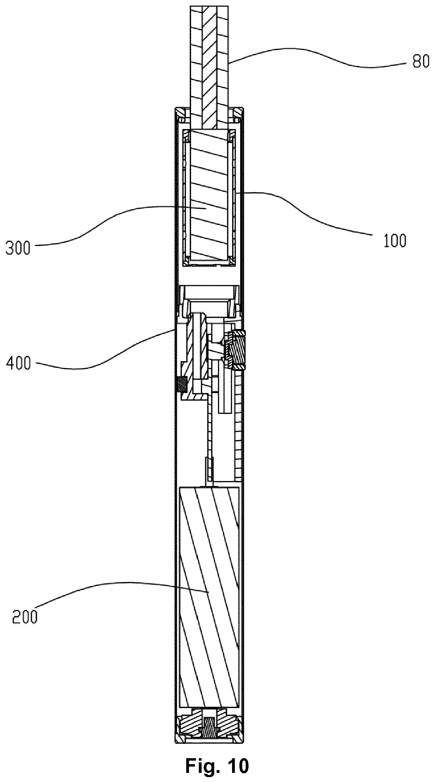

As shown in Figs. 1, 2, and 3, the example discloses a smoke generator 100 for assembling a smoke suction device in combination with the supply power source 200 (as shown in Fig. 10). The smoke generator comprises a supporting pipe 10 made of heat resistant materials, a plurality of heating bodies 60 having a first end 63 and a second end 64 that are disposed in the supporting pipe, a first support 20 having a gas outlet 21 and a second support 30 having a gas inlet 31, the first support 20 and the second support 30 being disposed on the two ends of the supporting pipe, wherein both the first support and the second support are installed with a plurality of positioning portions. The first end of the plurality of heating bodies is disposed on the first support and bent onto the positioning portion of the first support, and the second end is disposed on the second support and bent onto the positioning portion of the second support. The heating bodies 60 surrounds a cavity 11 that is adapted to be filled with solid smoking material 300. When the smoke generator 100 is in operation, the gas stream flows into the cavity 11 via the gas inlet 31, and discharged from the cavity 11 via the gas outlet 21.

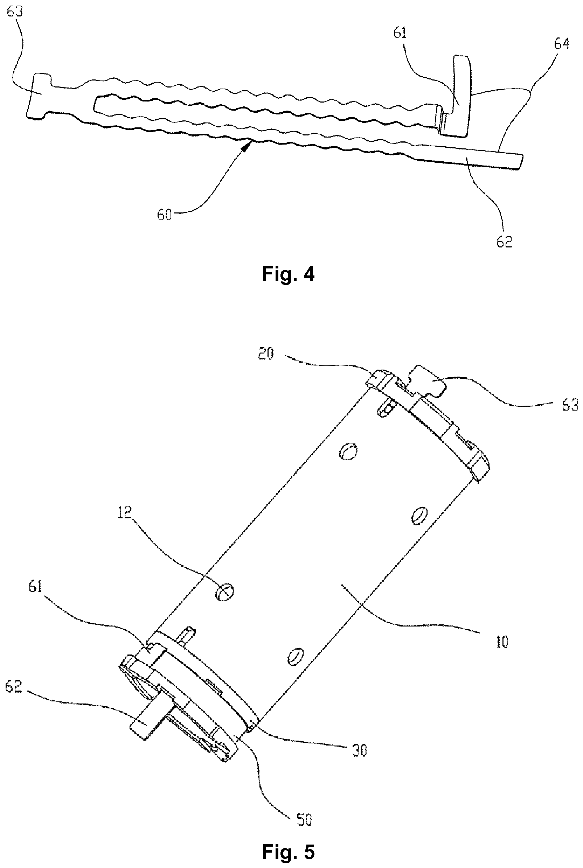

As shown in Fig. 4, in the example, the heating bodies 60 are in a sheet form, and the number of the heating bodies 60 is set to be five. The second ends 64 of the individual heating bodies 60 are divided into first pins 61 and second pins 62 adaptable for electrical connection to the electrodes of external supply power source 200, and the individual first pins 61, after being bent, are positioned onto the first support 20 and the individual first pins 61 are contacted with each other to form electrical connections.

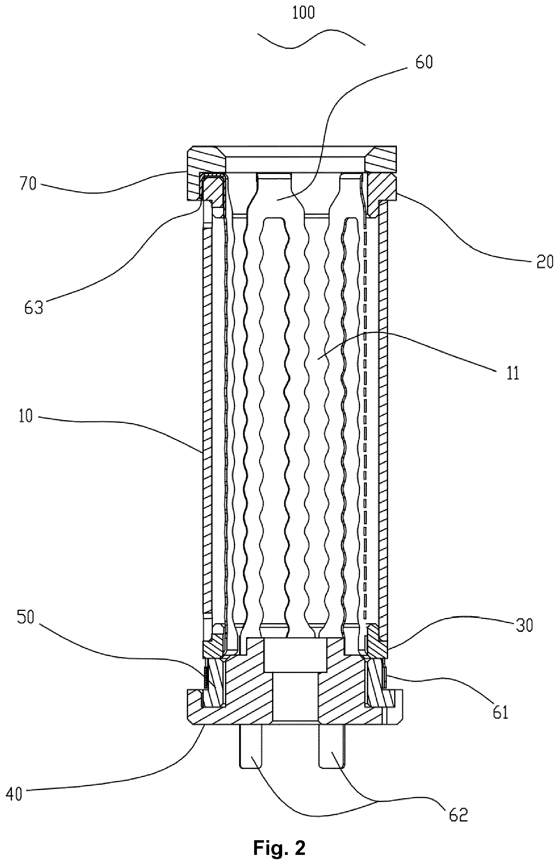

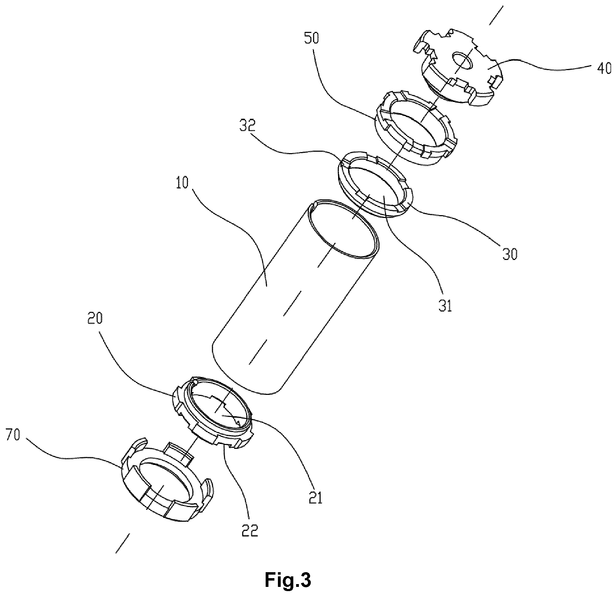

Further, as shown in Figs. 2 and 3, the smoke generator 100 further comprises a non-conductive fastener 50 that is partially fastened in the gas inlet 31, and the fastener 50 does not block the gas inlet 31. The fastener 50 is used to fix the individual fist pins 61 and separate the first pins 61 from the second pins 62 to prevent false contact conduction between the first pins 61 and the second pins 62 upon installation that can result in the rejection of the finished products. In the example, the fastener 50 is engaged with the base 40 on its outer surface, and the base 40 will not block the gas inlet 31. The base is used to fix the individual second pins 62 between the fastener 50 and the base 40. In other examples, the base 40 and the fattener 50 may be connected by the means of the sleeving connection.

As shown in Fig. 3, both the first support 20 and the second support 30 are installed with a plurality of positioning portions for fixing the first end 63 and the second end 64, and the first end 63 and second end 64 of the heating bodies 60 are bent onto the positioning portions of the first support 20 and the second support 30 respectively. In the example, the first support 20 is installed with a groove 22 adapted to contain the bent terminal portion of the first end 63 of the heating bodies 60 thereon, and the second support 30 is installed with a groove 32 adapted to contain the bend terminal portion of the first pins 61 of the heating bodies 60 thereon. Due to the installation regarding the groove 22 on the first support 20 and the groove 32 on the second support 30, after the heating bodies 60 are bent onto the first support 20 and the second support 30, it can prevent the movement of the individual heating bodies 60 upon assembly, and assure the prepared finished products to have beneficial effect that a stable smoke quantity can be persistently produced.

In order to further fix the heating bodies 60, as shown in Figs. 1 and 2, the smoke generator 100 further comprises a fixing cap 70 that is engaged at the periphery of the first support 20, and the fixing cap 70 will not block the gas outlet 70. The fixing cap 70 presses onto the first end 63 of the heating bodies 60 to fix it.

In the example, the supporting pipe 10 is made of a metallic material. The heating bodies 60 is supported by the first support 20 and the second support 30, and it is not in electric contact with the supporting pipe 20. In other embodiments, the supporting pipe 10 also can be made of ceramics and other heat resistant materials.

As shown in Figure 5, a plurality of through holes 12 are further disposed on the side of the supporting pipe 10, and the through holes 12 can pass through the cavity 11. The through holes 12 can be used for air entrance and have the same functions as the gas inlet 31. The objective of the through holes is to render the entrance of gases to the cavity 11 to be more gentle and uniform. When the heating bodies 60 heat the smoking material 300, smoke may be more quickly extracted.

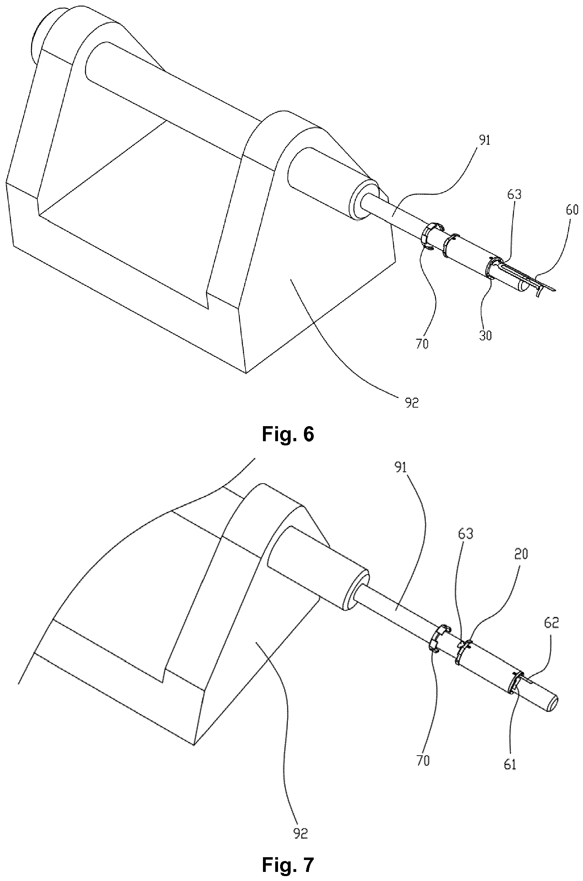

Figs. 6, 7, 8, and 9 are schematic views for illustrating the method for assembling the smoke generator 100. In the method, an assembling tool is usually used, and the assembling tool primarily comprises a substrate 92 and an assembling rod 91 disposed on the substrate 92. The size of the assembling rod 91 is adapted to the size of the cavity 11. Particularly, the assembling method comprises the steps:
a). fixing a first support 20 and a second support 30 respectively at two ends of a supporting pipe 10;
b). sleeving a fixing cap 70 onto an assembling rod 91 that is adapted to pass through the supporting pipe 10;
c). sleeving the supporting pipe 10 obtained in the step a) onto the assembling rod 91, and engaging the fixing cap 70 onto the first support 20 along the assembling rod 91;
d). inserting a first end 63 of the heating bodies 60 into a gap between the supporting pipe 10 and the assembling rod 91 along the end of the second support 30 until the first end 63 of the heating bodies 60 extends out of the end of the first support 20;
e). respectively bending two ends of the heating bodies 60 onto positioning portions of the first support 20 and second support 30 to fix the healing bodies 60; and
f). successively fixing the individual heating bodies 60 along the circumferential direction of the supporting pipe 10 onto the first support 30 and the second support 30, so that the plurality of heating bodies 60 surround a cavity 11 that is adapted to be filled with solid smoking materials, wherein the first pins 61 of the second end 64 of the individual heating bodies 60, after being bent, are positioned on the positioning portions of the second support 30 and the plurality of first pins 61 are contacted with each other, and the second pins 62 are intermittently fixed between the fastener 50 and the base 40, and pressing the fixing cap 70 onto the first end 63 of the heating bodies 60.

For example, the smoke generator 100 as shown in Fig. 1 comprises five heating bodies 60, a fixing cap 70, a fastener 50 and a base 40. Hence, after the step c) is completed, the five heating bodies 60, according to the step d), are assembled onto the supporting pipe 10 one by one; then according to the step e), the first ends 63 of the five heating bodies 60 are bent into the groove 22 one by one, and then the first pins 61 on the second end 64 are bent into the groove 32 one by one.

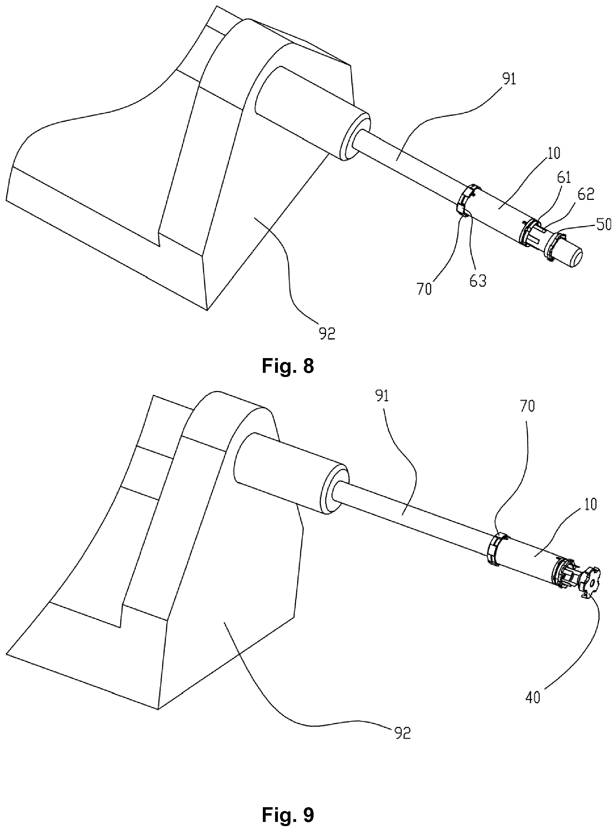

At last, as shown in Figure 8, the fastener 50 is sleeved onto the assembling rod 91, and a part of the fastener 50 is installed into the gas inlet 31. Further, as shown in Figure 9, the base 40 engages the outer surface of the fattener 50.

In the example, the solid smoking material 300 is solid tobacco. In other examples, the solid smoking material 300 may further be tobacco paste or herb.

### Example 2

The assembling method comprises the steps:
a). fixing a first support 20 and a second support 30 at two ends of a supporting pipe 10 respectively;
b). sleeving the supporting pipe 10 obtained in the step a) onto an assembling rod 91 adapted to pass through the supporting pipe 10;
c). inserting a first end 63 of the heating bodies 60 into a gap between the supporting pipe 10 and the assembling rod 91 along the end of the second support 30 until the first end 63 of the heating bodies 60 extends out of the end of the first support 20;
d). respectively bending two ends of the heating bodies 60 onto positioning portions of the first support 20 and second support 30 to fix the heating bodies 60;
e). successively fixing the individual heating bodies 60 along the circumferential direction of the supporting pipe 10 onto the first support 20 and the second support 30, so that the plurality of heating bodies 60 surround a cavity 11 that is adapted to be filled with solid smoking materials, wherein the first pins 61 of the second end 64 of the individual heating bodies 60, after being bent, are positioned on the positioning portions of the second support 30 and the plurality of first pins 61 are contacted with each other, and the second pins 62 are intermittently fixed between the fastener 50 and the base 40; and
f). detaching the supporting pipe 10, further sleeving the supporting pipe 10 onto the assembling rod 91 after the fixing cap 70 is sleeved onto the assembling rod 91, engaging the fixing cap 70 onto the first support 20 along the assembling rod 91 and pressing the fixing cap 70 onto the first end 63 of the heating bodies 60.

The above specific examples are used to illustrate the principles and embodiments of the invention. It should be understood that the above embodiments are only used for aiding the understandings to the invention, and they cannot be understood to be restrict the invention. For a person skilled in the art, according to the concept of the invention, any slight variations or equivalent substitutions to the structural shape or configuration of the invention should be contained in the claimed protection scope.

## Claims

1. A smoke generator, the smoke generator (100) comprises: a supporting pipe (10), a plurality of heating bodies (60) having a first end (63) and a second end (64) that are disposed in the supporting pipe (10), **characterized in that** a first support (20) having an gas outlet (21) and a second support (30) having a gas inlet (31), the first support (20) and the second support (30) being respectively disposed at the two ends of the supporting pipe (10), and a plurality of positioning portions being disposed onto the first support (20) and the second support (30), wherein the first end (63) of each of the plurality of heating bodies (60) is disposed onto the first support (20) and bent onto the positioning portions of the first support (20), the plurality of heating bodies (60) surrounds a cavity (11) that is adapted to be filled with smoking materials, **characterized in that** the second end (64) of each of the plurality of heating bodies (60) is disposed on the second support (30);the second end (64) of the heating bodies (60) includes a first pin (61) and a second pin (62), in which the second pin (62) extends out of the second support (30), and the first pin (61) is bent into an arc and then fixed onto the positioning portions of the second support (30).

2. The smoke generator according to claim 1, **characterized in that** a plurality of through holes (12) for gas entrance are disposed onto the sidewalls of the supporting pipe (10).

3. A method for assembling a smoke generator, wherein the smoke generator (100) comprises: a supporting pipe (10), a plurality of heating bodies (60) having a first end (63) and a second end (64) that are disposed in the supporting pipe (10), a first support (20) having an gas outlet (21) and a second support (30) having a gas inlet (31), the first support (20) and the second support (30) being respectively disposed at the two ends of the supporting pipe (10), and a plurality of positioning portions being disposed onto the first support (20) and the second support (30), wherein the first end (63) of each of the plurality of heating bodies (60) is disposed onto the first support (20) and bent onto the positioning portions of the first support (20), and the second end (64) of each of the plurality of heating bodies (60) is disposed on the second support (30) and bent onto the positioning portions of the second support (30); the second end (64) of each heating body (60) of the smoke generator (100) is divided into a first pin (61) and a second pin (62) that are adaptable for electrical connection to the electrodes of the external supply power source, and the smoke generator (100) further comprises a non-conductive fastener (50) that is fastened in the gas inlet (31), and a base (40) that is engaged or sleeved onto the fastener (50); and the plurality of heating bodies (60) surrounds a cavity (11) that is adapted to be filled with smoking materials, **characterized in that**, the method for assembling the smoke generator comprises the steps of:
a). fixing a first support (20) and a second support (30) at two ends of a supporting pipe (10) respectively;
b). sleeving the supporting pipe (10) obtained in the step a) onto an assembling rod (91) adapted to pass through the supporting pipe (10);
c). inserting a first end (63) of the heating bodies (60) into a gap between the supporting pipe (10) and the assembling rod (91) along the end of the second support (30) until the first end (63) of the heating bodies (60) extends out of the end of the first support (20);
d). respectively bending two ends of the heating bodies (60) onto positioning portions of the first support (20) and second support (30) to fix the heating bodies (60); and the first pin (61) of the second end (64) of each heating body (60), after being bent, is positioned onto the position portion of the second support (30), and the plurality of the first pins (61) are brought into contact with each other, and the individual second pins (62) are intermittently fixed between the fastener (50) and the base (40);
e). successively fixing the individual heating bodies (60) along the circumferential direction of the supporting pipe (10) onto the first support (20) and the second support (30), so that the plurality of heating bodies (60) surround a cavity (11) that is adapted to be filled with solid smoking materials.

4. The method for assembling a smoke generator according to claim 3, **characterized in that** the assembling rod (91) is a part of an assembling tool, and the assembling tool comprises a substrate (92) and an assembling rod (91) disposed on the substrate (92).

5. The method for assembling a smoke generator according to claim 3, **characterized in that** the positioning portion of the first support (20) or the second support (30) is a groove that is adapted to contain the bent first end (63) or bent second end (64) of the heating bodies (60).

6. The method for assembling a smoke generator according to claim 3, **characterized in that** the smoke generator (100) further comprises a fixing cap (70) that engages the first support (20), and the method for assembling the smoke generator further comprises the step:
f). detaching the supporting pipe (10), further sleeving the supporting pipe (10) onto the assembling rod (91) after the fixing cap (70) is sleeved onto the assembling rod (91), and engaging the fixing cap (70) onto the first support (20) along the assembling rod (91) while the fixing cap (70) is pressured onto the first end (63) of the heating bodies (60).

## Patentansprüche

1. Ein Raucherzeuger, der Raucherzeuger (100) umfassend: ein Halterohr (10), eine Vielzahl an Heizelementen (60) mit einen ersten Ende (63) und einem zweiten Ende (64), welche in dem Halterohr (10) angeordnet sind, **dadurch gekennzeichnet, dass** eine erste Halterung (20) mit einem Gasauslass (21) und eine zweite Halterung (30) mit einem Gaseinlass (31), wobei die erste Halterung (20) und die zweite Halterung (30) jeweils an den zwei Enden des Halterohrs (10) angeordnet sind, und eine Vielzahl an Positionierabschnitten auf der ersten Halterung (20) und der zweiten Halterung (30) angeordnet sind, wobei das erste Ende (63) jedes der vielzähligen Heizelemente (60) auf der ersten Halterung (20) angeordnet und auf die Positionierabschnitte an der ersten Halterung (20) gebogen wird, die Vielzahl der Heizelemente (60) einen Hohlraum (11) umgeben, der für die Befüllung mit Rauchmaterialien geeignet ist, **gekennzeichnet dadurch, dass** das zweite Ende (64) jedes der vielzähligen Heizelemente (60) auf der zweiten Halterung (30) angeordnet ist; das zweite Ende (64) der Heizelemente (60) einen ersten Kontakt (61) und einen zweiten Kontakt (62) beinhaltet, wobei der zweite Kontakt (62) aus der zweiten Halterung (30) herausragt, und der erste Kontakt (61) bogenförmig gebogen ist und auf den Positionierabschnitten der zweiten Halterung (30) befestigt ist.

2. Der Raucherzeuger gemäß Anspruch 1, **dadurch gekennzeichnet, dass** eine Vielzahl an Durchlassöffnungen (12) zum Gaseintritt auf den Seitenwänden des Halterohres (10) angeordnet sind.

3. Ein Verfahren zur Montage eines Raucherzeugers, der Raucherzeuger (100) umfassend: ein Halterohr (10), eine Vielzahl an Heizelementen (60) mit einen ersten Ende (63) und einem zweiten Ende (64), welche in dem Halterohr (10) angeordnet sind, eine erste Halterung (20) mit einem Gasauslass (21) und eine zweite Halterung (30) mit einem Gaseinlass (31), wobei die erste Halterung (20) und die zweite Halterung (30) jeweils an den zwei Enden des Halterohrs (10) angeordnet ist, und eine Vielzahl an Positionierabschnitten auf der ersten Halterung (20) und der zweiten Halterung (30) angeordnet ist, wobei das erste Ende (63) jedes der vielzähligen Heizelemente (60) auf der ersten Halterung (20) angeordnet ist und auf die Positionierabschnitte an der ersten Halterung (20) gebogen ist, und das zweite Ende (64) jedes der vielzähligen Heizelemente (60) auf der zweiten Halterung (30) angeordnet ist und auf die Positionierabschnitte der zweiten Halterung (30) gebogen ist; das zweite Ende (64) jedes Heizelements (60) des Raucherzeugers (100) in einen ersten Kontakt (61) und einen zweiten Kontakt (62) unterteilt ist, welche zur elektrischen Kontaktierung zu den Elektroden der externen Versorgerstromquelle konfigurierbar sind, und der Raucherzeuger (100) weiterhin eine nichtleitende Befestigung (50), welche in dem Gaseinlass (31) befestigt ist, und eine Basis (40) enthält, welche die Befestigung (50) umgreift oder darüber gezogen ist; und die Vielzahl der Heizelemente (60) einen Hohlraum (11) umgibt, der für die Befüllung mit Rauchmaterialien geeignet ist, **dadurch gekennzeichnet, dass** das Verfahren zur Montage des Raucherzeugers die folgenden Schritte umfasst:
a). Befestigen einer ersten Halterung (20) und einer zweiten Halterung (30) an jeweils zwei Enden eines Halterohres;
b). Überziehen des in dem Schritt a) erhaltenen Halterohres (10) auf eine zum Durchführen durch das Halterohr (10) konfigurierten Montagestange (91);
c). Einführen eines ersten Endes (63) der Heizelemente (60) in eine Lücke zwischen dem Halterohr (10) und der Montagestange (91) entlang des Endes der zweiten Halterung (30), bis das erste Ende (63) der Heizelemente (60) aus dem Ende der ersten Halterung (20) herausragt;
d). jeweils Biegen der zwei Enden der Heizelemente (60) auf Positionierabschnitte der ersten Halterung (20) und zweiten Halterung (30) um die Heizelemente (60) zu befestigen; und der erste Kontakt (61) des zweiten Endes (64) jedes Heizelementes (60) nach dem Biegen auf dem Positionierabschnitt der zweiten Halterung (30) angeordnet wird, und die Vielzahl der ersten Kontakte (61) miteinander in Kontakt gebracht werden, und die einzelnen zweiten Kontakte (62) intermittierend zwischen der Befestigung (50) und der Basis (40) befestigt werden;
e). nacheinander Befestigen der einzelnen Heizelemente (60) entlang der umlaufenden Richtung des Halterohres (10) auf die erste Halterung (20) und die zweite Halterung (30), sodass die vielzähligen Heizelemente (60) einen Hohlraum (11) umgeben, der dazu geeignet ist, mit festen Rauchmaterialien befüllt zu werden.

4. Das Verfahren zur Montage eines Raucherzeugers gemäß Anspruch 3, **dadurch gekennzeichnet, dass** die Montagestange (91) ein Teil eines Montagewerkzeuges ist, und das Montagewerkzeug einen Träger (92) und eine auf dem Träger (92) angeordnete Montagestange (91) umfasst.

5. Das Verfahren zur Montage eines Raucherzeugers gemäß Anspruch 3, **dadurch gekennzeichnet, dass** der Positionierabschnitt der ersten Halterung (20) oder der zweiten Halterung (30) eine Nut ist, die dazu geeignet ist, das gebogene erste Ende (63) oder das gebogene zweite Ende (64) der Heizelemente (60) zu beinhalten.

6. Das Verfahren zur Montage eines Raucherzeugers gemäß Anspruch 3, **dadurch gekennzeichnet, dass** der Raucherzeuger (100) weiterhin eine Befestigungskappe (70) umfasst, welche die erste Halterung (20) umgreift, und das Verfahren zur Montage des Raucherzeugers weiterhin den folgenden Schritt umfasst: f). Lösen des Halterohres (10), weiterhin Überziehen des Halterohres (10) auf die Montagestange (91), nachdem die Befestigungskappe (70) auf die Montagestange (91) übergezogen wird, und Einrasten der Befestigungskappe (70) auf die erste Halterung (20) entlang der Montagestange (91), während die Befestigungskappe (70) auf das erste Ende (63) der Heizelemente (60) gedrückt wird.

## Revendications

1. Générateur de fumées, le générateur de fumées (100) comprend : un tuyau de support (10), une pluralité de corps de chauffe (60) ayant une première extrémité (63) et une seconde extrémité (64) qui sont disposées dans le tuyau de support (10), **caractérisé par** un premier support (20) ayant une sortie de gaz (21) et un second support (30) ayant une entrée de gaz (31), le premier support (20) et le second support (30) étant respectivement disposés sur les deux extrémités du tuyau de support (10), et une pluralité de parties de positionnement étant disposées sur le premier support (20) et le second support (30), dans lequel la première extrémité (63) de chacun de la pluralité de corps de chauffe (60) est disposée sur le premier support (20) et pliée sur les parties de positionnement du premier support (20),
la pluralité de corps de chauffe (60) entoure une cavité (11) qui est adaptée pour être remplie de matériaux de fumée, **caractérisé en ce que** la seconde extrémité (64) de chacune de la pluralité de corps de chauffe (60) est disposée sur le second support (30) ; la seconde extrémité (64) des corps de chauffe (60) inclut une première broche (61) et une seconde broche (62) dans lequel la seconde broche (62) s'étend hors du second support (30), et la première broche (61) est pliée en un arc puis fixée sur les parties de positionnement du second support (30).

2. Générateur de fumées selon la revendication 1, **caractérisé en ce qu'**une pluralité de trous traversants (12) pour l'entrée du gaz sont disposées sur les parois latérales du tuyau de support (10).

3. Procédé destiné à l'assemblage d'un générateur de fumées, dans lequel le générateur de fumées (100) comprend : un tuyau de support (10), une pluralité de corps de chauffe (60) ayant une première extrémité (63) et une seconde extrémité (64) qui sont disposées dans le tuyau de support (10), un premier support (20) ayant une sortie de gaz (21) et un second support (30) ayant une entrée de gaz (31), le premier support (20) et le second support (30) étant respectivement disposés sur les deux extrémités du tuyau de support (10), et une pluralité de parties de positionnement étant disposées sur le premier support (20) et le second support (30), dans lequel la première extrémité (63) de chacun de la pluralité de corps de chauffe (60) est disposée sur le premier support (20) et pliée sur les parties de positionnement du premier support (20), et la seconde extrémité (64) de chacun de la pluralité de corps de chauffe (60) est disposée sur le second support (30) et pliée sur les parties de positionnement du second support (30) ; la seconde extrémité (64) de chaque corps de chauffe (60) du générateur de fumées (100) est divisée en une première broche (61) et une seconde broche (62) qui sont adaptables pour la connexion électrique aux électrodes de la source de l'électricité d'alimentation extérieure, et le générateur de fumées (100) comprend en outre une fixation non-conductrice (50) qui est fixée dans l'entrée de gaz (31), et une base (40) qui est engagée ou manchonnée sur la fixation (50) ; et la pluralité de corps de chauffe (60) entoure une cavité (11) qui est adaptée pour être remplie avec des matériaux de fumée, **caractérisé en ce que** le procédé pour assembler le générateur de fumées comprend les étapes de :
a) fixer un premier support (20) et un second support (30) à deux extrémités d'un tuyau de support (10) respectivement ;
b) manchonner le tuyau de support (10) obtenu à l'étape a) sur une tige d'assemblage (91) adaptée pour passer au travers du tuyau de support (10) ;
c) insérer une première extrémité (63) des corps de chauffe (60) dans un espace entre le tuyau de support (10) et la tige d'assemblage (91) le long de l'extrémité du second support (30) jusqu'à ce que la première extrémité (63) des corps de chauffe (60) s'étende hors de l'extrémité du premier support (20) ;
d) plier respectivement deux extrémités des corps de chauffe (60) sur des parties de positionnement du premier support (20) et du second support (30) pour fixer les corps de chauffe (60) ; et la première broche (61) de la seconde extrémité (64) de chaque corps de chauffe (60), après avoir été pliée, est positionnée sur la partie de positionnement du second support (30), et la pluralité des premières broches (61) est mise en contact l'une avec l'autre, et les secondes broches (62) individuelles sont fixées de manière intermittente entre la fixation (50) et la base (40) ;
e) fixer successivement les corps de chauffe (60) individuels le long de la direction circonférentielle du tuyau de support (10) sur le premier support (20) et le second support (30) de façon à ce que la pluralité de corps de chauffe (60) entoure une cavité (11) qui est adaptée pour être remplie de matériaux de fumée solides.

4. Procédé destiné à l'assemblage d'un générateur de fumées selon la revendication 3, **caractérisé en ce que** la tige d'assemblage (91) fait partie d'un outil d'assemblage, et l'outil d'assemblage comprend un substrat (92) et une tige d'assemblage (91) disposée sur le substrat (92).

5. Procédé destiné à l'assemblage d'un générateur de fumées selon la revendication 3, **caractérisé en ce que** la partie de positionnement du premier support (20) ou du second support (30) est une rainure qui est adaptée pour contenir la première extrémité (63) pliée ou seconde extrémité (64) pliée des corps de chauffe (60).

6. Procédé destiné à l'assemblage d'un générateur de fumées selon la revendication 3, **caractérisé en ce que** le générateur de fumées (100) comprend en outre un bouchon de fixation (70) qui engage le premier support (20), et le procédé pour assembler le générateur de fumées comprend en outre l'étape de :
f) détacher le tuyau de support (10), manchonner ensuite le tuyau de support (10) sur la tige d'assemblage (91) après que le bouchon de fixation (70) est manchonné sur la tige d'assemblage (91), et engager le bouchon de fixation (70) sur le premier support (20) le long de la tige d'assemblage (91) tandis que le bouchon de fixation (70) est comprimé sur la première extrémité (63) des corps de chauffe (60).
